# FASCICULE DE BREVET EUROPEEN

(11) **EP 2 983 685 B1**
(45) Date de publication et mention de la délivrance du brevet: **21.11.2018**
(21) Numéro de dépôt: 14718542.5
(22) Date de dépôt: 08.04.2014
(51) Int. Cl.: A61K 36/35, A61K 35/74, A61P 31/12

(54) **COMPOSITION COMPRENANT UNE ASSOCIATION D'UN EXTRAIT DE SUREAU ET D'UNE SOUCHE DE LACTOBACILLUS RHAMNOSUS**
VERWENDUNG EINER KOMBINATION AUS EINEM HOLLUNDEREXTRAKT UND LACTOBACILLUS RHAMNOSUS-STAMM
COMPOSITION COMPRISING A COMBINATION OF AN ELDER EXTRACT AND A STRAIN OF LACTOBACILLUS RHAMNOSUS

(30) Priorité: 09.04.2013 FR 1353193
(43) Date de publication de la demande: 17.02.2016
(73) Titulaire: Pierre Fabre Médicament, 92100 Boulogne-Billancourt (FR)
(72) Inventeur: LATGE, Christian Jacques, F-31130 Balma (FR); LIBON, Christine, F-31320 Castanet-Tolosan (FR)
(74) Mandataire: Regimbeau
(86) Numéro de dépôt international: PCT/EP2014/057082
(87) Numéro de publication internationale: WO 2014/166960

(56) Documents cités:
- WO-A1-2010/132017
- DATABASE GNPD [Online] MINTEL; juillet 2011 (2011-07), "Viral Guard Fizzi Chews", XP002715502, Database accession no. 1576267
- DATABASE GNPD [Online] MINTEL; septembre 2005 (2005-09), "Viral Guard Cold & Flu Immune System Booster", XP002715503, Database accession no. 400294
- FRØKIAER HANNE ET AL: "Astragalus root and elderberry fruit extracts enhance the IFN-[beta] stimulatory effects of Lactobacillus acidophilus in murine-derived dendritic cells.", PLOS ONE 2012, vol. 7, no. 10, 2012, page e47878, XP009173804, ISSN: 1932-6203
- Anonyme: "Sambucus nigra Monograph", Alternative Medicine Review , vol. 10, no. 1 2005, pages 51-55, XP002715504, Extrait de l'Internet: URL:http://www.thorne.com/altmedrev/.fullt ext/10/1/51.pdf [extrait le 2013-10-28]
- LEE YU-NA ET AL: "Sublingual administration of Lactobacillus rhamnosus affects respiratory immune responses and facilitates protection against influenza virus infection in mice", ANTIVIRAL RESEARCH, vol. 98, no. 2, 21 mars 2013 (2013-03-21), pages 284-290, XP002715505,
- Inês J. Seabra & Mara E. M. Braga &Maria T. P. Batista & Hermínio C. de Sousa: "Fractioned High Pressure Extraction of Anthocyaninsfrom Elderberry (Sambucus nigra L.) Pomace", Food Bioprocess Technol , 2008, XP002715506, Extrait de l'Internet: URL:https://estudogeral.sib.uc.pt/bitstrea m/10316/7670/1/obra.pdf [extrait le 2013-10-28]

## Description

La présente invention concerne une composition comprenant une association d'un extrait de sureau et d'au moins une souche de *Lactobacillus rhamnosus*, destinée à stimuler l'immunité et/ou à favoriser une réponse anti-infectieuse et/ou anti-inflammatoire.

Le sureau ou *Sambucus nigra L*. appartient à la famille des *Adoxaceae*. Le genre *Sambucus* comprend 25 espèces distribuées mondialement dans les régions tempérées. Le sureau noir, *S. nigra ssp. nigra*, est largement cultivé en Europe, notamment au Danemark en Italie et en Autriche, dans la région nordique de l'Afrique et dans la partie ouest de l'Asie. Le sureau du Canada, *S. nigra ssp. canadensis*, est originaire du nord-est de l'Amérique du Nord et cultivé dans l'Oregon, en Pennsylvanie et au Kansas, malgré une sélection réalisée au Canada.

Les différents organes du sureau : écorces, racines, tiges, fleurs, feuilles et fruits sont utilisés traditionnellement dans des domaines aussi divers que la médecine, l'alimentation et la fabrication d'outils et de jouets. Les productions industrielles les plus importantes concernent de nos jours la production d'extraits de baies de sureau à destination des marchés nutraceutiques et de l'industrie des colorants.

Certaines publications font l'état de l'effet d'un extrait de baies de sureau sur la production de cytokines (Barak et al., European Cytokine network, Vol 12(2), 290-296, 2001). Il a notamment déjà été démontré l'effet d'un extrait de sureau sur la diminution des symptômes liés à une infection grippale due à des virus Influenza (A et/ou B et/ou C) ainsi que son effet antiviral sur les virus de type 1 de l'herpes simplex et le virus respiratoire syncytial (Zakay-Rones et al., The Journal of International Medical Research, vol 32, 132-140, 2004).

De très nombreuses études scientifiques ont démontré les effets bénéfiques sur la santé de certains microorganismes présents dans des aliments fermentés, notamment des produits laitiers. Ces microorganismes sont communément dénommés « probiotiques ». Selon la définition généralement admise à l'heure actuelle, les probiotiques sont : des microorganismes vivants, qui lorsqu'ils sont consommés en quantités adéquates, ont un effet bénéfique sur la santé de l'hôte (rapport OMS sur l'évaluation des propriétés sanitaires et nutritionnelles des probiotiques dans les aliments, y compris le lait en poudre contenant des bactéries lactiques vivantes, Cordoba, Argentine, 1-4 octobre 2001).

Il a été montré dans les demandes de brevets WO96/20607, EP0794707, EP1283714 et FR292912657 que la consommation de produits alimentaires contenant des bactéries probiotiques peut produire des effets favorables sur la santé, par l'intermédiaire notamment du rééquilibrage de la flore intestinale, de l'amélioration de la résistance aux infections, et de la modulation de la réponse immunitaire.

Les microorganismes probiotiques utilisés en alimentation humaine sont généralement des bactéries lactiques, appartenant principalement aux genres *Lactobacillus* et *Bifidobacterium* et notamment à l'espèce *Lactobacillus paracasei*.

*Lactobacillus* est un genre de bactéries à gram positif, immobiles, de formes et dimensions variables anaérobies facultatives. Il est appelé ainsi car la plupart de ses membres convertissent le lactose et d'autres sucres simples en acide lactique. Chez l'homme, les lactobacilles sont des hôtes très répandus comme commensaux et généralement utiles, voire nécessaires. Ils constituent un élément important de la flore intestinale.

*Lactobacillus rhamnosus* est une bactérie qui a été initialement considérée comme uns sous-espèce de *lactobacillus casei*, mais la recherche génétique a prouvé que c'était une espèce à part entière. *Lactobacillus rhamnosus* se trouve normalement dans l'estomac et dans le tube digestif. Cette bactérie a des propriétés qui sont bénéfiques au tractus intestinal, mais également pour le système immunitaire en particulier dans la lutte contre les agents pathogènes des voies intestinales et urinaires.

Les probiotiques peuvent exercer des effets directs sur le chyme, la flore, on parle d'effets luminaux, ou au niveau des entérocytes ou des cellules immunocompétentes du Galt, on parle alors d'effets pariétaux. Ils peuvent aussi avoir des effets indirects liés aux modifications de l'écosystème ou du système immunitaire local.

Certains probiotiques ont une capacité d'adhérence à l'épithélium digestif. Cette propriété peut constituer un avantage écologique favorisant les chances d'interrelations étroites avec l'épithélium entérocytaire et le système immunitaire local. Une étude a montré qu'une souche adhérente de *Lactobacillus rhamnosus* pouvait coloniser de manière prolongée la muqueuse jéjunale et/ou rectale chez quelques sujets (Alander et al., Letters in Applied Microbiology, 24 (5), vol 361-364, 1997) .

Des travaux de plus en plus nombreux démontrent des effets biologiques de multiples souches, il est important de noter que ces effets apparaissent souche-dépendants.

La demande de brevet US 2006/0233895 décrit une composition comprenant un extrait de sureau qui peut éventuellement être associé avec un probiotique tel que le *Lactobacillus casei*. Toutefois, cette composition contient obligatoirement un mélange d'Uncaria Tomentosa, de Pau d'Arco, de Scrutellaria Baicalensis et d'Artemisine. Par ailleurs, cette composition n'est pas destinée à stimuler l'immunité mais sert à traiter la maladie de Lyme qui est causée par une infection bactérienne et qui est donc très différente des infections par les virus du type influenza.

La demande de brevet WO 2010/043696 divulgue une composition comprenant une combinaison d'un extrait de sureau noir et d'une souche de *Lactobacillus paracasei, Lactobacillus casei, Lactobacillus bulgaricus*, ou de *Streptococcus thermophilus* destinée à stimuler l'immunité. Dans cette demande, une synergie entre le sureau et *Lactobacillus paracasei* est démontrée pour la production d'IL-10 et d'interféron-gamma. Toutefois la limite de la réaction inflammatoire n'est pas connue. Des données sur les autres cytokines et chemokines du type CXCL10 lèveraient le risque d'une augmentation trop importante de cette réponse inflammatoire.

La présente invention est définie dans les revendications et concerne donc une association synergique comprenant un extrait de sureau et au moins une souche de *Lactobacillus rhamnosus.*

La composition comprenant un extrait de sureau et au moins une souche de *Lactobacillus rhamnosus* peut en outre comprendre des vitamines et/ou des sels minéraux.

Dans un mode de réalisation particulier, la composition peut être préparée par le procédé comprenant les étapes suivantes :
- préparation de l'association ;
- ajout de l'association dans la composition selon la présente invention.

Dans ce cas, l'association est donc préparée avant son ajout dans la composition. Toutefois il est également possible de préparer une composition par ajout de chacun des constituants de l'association de manière séparée dans la composition, c'est à dire sans préparer l'association au préalable.

Préférentiellement, la composition est destinée à l'administration par voie orale.

Avantageusement la composition est un aliment, un complément alimentaire, un médicament ou un produit OTC (Over The Counter).

Avantageusement, l'extrait de sureau est obtenu à partir des baies et/ou fleurs de sureau, et préférentiellement à partir des baies de sureau. De façon avantageuse, l'extrait de sureau selon la présente invention est un extrait hydrosoluble.

L'extrait de sureau utilisé dans le cadre de la présente invention peut être caractérisé par sa teneur d'une part en anthocyanes et d'autre part en grosses molécules telles que des protéines (en particulier des lectines). Les anthocyanes sont des pigments naturels des feuilles, des pétales et des fruits, situés dans les vacuoles des cellules, solubles dans l'eau, allant du rouge orangé au bleu pourpre dans le spectre visible.

Avantageusement, l'extrait de sureau comprend des anthocyanes, de façon avantageuse de la famille des pelargonidines et des cyanidines, en une quantité comprise entre environ 0,5% et environ 25%, de façon avantageuse entre environ 3% et environ 25% et de façon encore plus avantageuse entre environ 8% et environ 16% en poids, par rapport à la matière sèche de l'extrait. De préférence, la teneur en anthocyanes est égale à environ 12% en poids, par rapport à la matière sèche de l'extrait. Ces teneurs sont exprimées en cyanidin-3- glucoside selon la méthode HPLC décrite par Wu et al., (J. Agric. Food Chem. 52(26), 7846-7856, 2004).

Avantageusement, l'extrait de sureau comprend des protéines en une quantité comprise entre 2 et 10% en poids, exprimés par rapport à la matière sèche (N x 6,25 selon la méthode de Kjeldahl : Protéines végétales, Coord. B. Godon, Collection Sciences et techniques agro-alimentaires, Technique et Documentation Lavoisier, Paris, 1985).

Dans le cadre de la présente invention, l'extrait de sureau est disponible dans le commerce ou peut être obtenu par un procédé comprenant les étapes suivantes :
Les baies de sureau fraîches ou congelées peuvent subir un pressage afin d'obtenir un jus après filtration. Il peut subir une étape d'hydrolyse enzymatique (pectinase ou mélange pectin méthylestérase et polygalacturonase) afin de le clarifier avant filtration (Girard and Fukumoto, Crit. Rev. Food Sci. Nutr. 40(2), 91-157, 2000). Une extraction aqueuse des baies séchées et broyées peut également être réalisée, suivie d'une séparation solide / liquide par filtration. Les extraits ainsi obtenus peuvent être utilisés tels quel ou concentrés, ou séchés sous forme de poudre. La teneur en anthocyanes, à ce stade, est comprise entre 0,5% et 3% en poids par rapport à la matière sèche de l'extrait, et avantageusement égale à environ 1% (dosage HPLC, exprimé en cyanidin-3-glucoside).

L'extrait de sureau peut être enrichi, en particulier en anthocyanes et/ou en molécules de haut poids moléculaires (protéines, polyphénols, polysaccharides). Pour cela, plusieurs méthodes bien connues de l'homme du métier sont possibles :
- Ultrafiltration : étape de diafiltration sur du jus préalablement dilué puis surconcentration sur membrane organique ou minérale avec un seuil de coupure compris entre 1 et 20kDa, et préférentiellement compris entre 3 et 10kDa (Girard and Fukumoto, Crit. Rev. Food Sci. Nutr. 40(2), 91-157, 2000).
- Passage sur colonne de résine polymérique absorbante de type Amberlite XAD.
- Extraction hydro-alcoolique (mélange en toutes proportions d'eau et d'alcool en C1 à C4) ou solutions aqueuses à pH acide (Bronnum-Hansen and Flink, Int. J. Food Sci. Tech. 21(2), 605-614, 1986 ; Lee and Wrolstad, J. Food Sci. 69(7), 564-573, 2004). Cette extraction peut être avantageusement réalisée directement sur les baies séchées et broyées ou sur de la purée réalisée à partir des baies fraîches.

L'extrait ainsi obtenu peut être concentré thermiquement (à une température n'excédant pas 50°C et sous vide) afin d'augmenter les degrés Brix et stabiliser vis à vis de contaminations microbiologiques. Il peut également être séché seul ou sur un support (par exemple maltodextrine, lactose...).

Le jus enrichi obtenu se situe préférentiellement entre 40 et 60° Brix, entre 3 et 25% d'anthocyanes et entre 5 et 10% de protéines (méthode de Kjeldahl, N x 6,25) exprimés en poids par rapport à la matière sèche.

Avantageusement, la quantité d'extrait sec de sureau par dose unitaire présente dans la composition, selon l'invention, est comprise entre environ 10 mg et environ 1 g. Plus avantageusement, la composition comprend entre environ 20 mg et environ 200 mg d'extrait sec de sureau par dose unitaire et de façon plus avantageuse, entre environ 20 et 100 mg d'extrait sec de sureau par dose unitaire, et de façon encore plus avantageuse entre 40 mg et 80 mg d'extrait sec de sureau par dose unitaire, et d'une autre manière tout aussi avantageuse entre 20 et 60 mg d'extrait sec de sureau par dose unitaire.

Dans le cadre de la présente invention, le terme « dose unitaire » représente la quantité de composition selon l'invention administrée en une seule prise. Avantageusement une dose unitaire selon la présente invention peut correspondre par exemple à un yaourt de 100 ou 125 ml, d'une gélule de taille classique ou à un comprimé de 2g. Ainsi, quel que soit le poids de la composition, la quantité d'extrait sec de sureau et/ou de souche de *Lactobacillus rhamnosus* par dose unitaire reste constante.

Une souche de *Lactobacillus rhamnosus* convenant tout particulièrement dans le cadre de la présente invention est la souche *Lactobacillus rhamnosus* GG (ATCC 53103). Cette souche de *Lactobacillus rhamnosus* est une souche isolée en 1983 dans le tractus intestinal chez l'homme en bonne santé. Cette souche fait l'objet du brevet US 4839281. La souche *Lactobacillus rhamnosus* GG s'est montrée bénéfique dans la prévention de différents types de diarrhées chez l'adulte et chez l'enfant (Guandalini et al., J. Pediatr. Gastroenterol. Nutr. 30(1), 54-60, 2000 ; Osterlund et al., Br. J. Cancer 97, 1028-1034, 2007). Il a également été rapporté que la souche *Lactobacillus rhamnosus* GG était à l'origine d'une réduction du risque d'infections du tractus respiratoire chez l'enfant (Hojsak et al., Clin. Nutr. 29(3), 312-316, 2010).

*Lactobacillus rhamnosus* est également d'une aide considérable pour le système immunitaire, en particulier dans la lutte contre les agents pathogènes des voies intestinales et urinaires. *Lactobacillus rhamnosus* se fixe à la muqueuse de l'intestin, où il encourage la croissance de bonnes bactéries qui aident à la digestion. Lactobacillus rhamnosus est une bactérie qui aide à éliminer et à prévenir la croissance de bactéries nocives dans les intestins.

Avantageusement, la composition comprendra 1.10⁷ à 1.10¹¹ UFC de *lactobacillus rhamnosus* par dose unitaire, préférentiellement 1.10⁸ à 1.10⁹ UFC par dose unitaire et de manière encore préférée 5.10⁸ UFC par dose unitaire.

Dans un mode de réalisation particulier de l'invention, la composition comprend, par dose unitaire,
- de 10 mg à 1 g d'extrait sec de sureau, préférentiellement de 20 mg à 200 mg, avantageusement de 20 mg à 100 mg et plus préférentiellement de 40 mg à 80 mg ; et d'une autre manière tout aussi avantageuse de 20 à 60 mg.
- De 1.10⁷ à 1.10¹¹ de *lactobacillus rhamnosus,* préférentiellement de 1.10⁸ à 1.10⁹ UFC par dose unitaire.

Dans un mode de réalisation particulier de l'invention, la composition comprend, par dose unitaire,
- de 2 mg à 200 mg d'anthocyanes de sureau, préférentiellement de 2 mg à 100 mg et plus préférentiellement de 2 mg à 20 mg, et d'une autre manière tout aussi avantageuse de 20 mg à 50mg.
- de 2 mg à 100 mg de protéines de sureau, préférentiellement de 10 mg à 20 mg ; et
- de 1.10⁷ à 1.10¹¹ de *lactobacillus rhamnosus,* préférentiellement de 1.10⁸ à 1.10⁹ UFC par dose unitaire.

On observe alors de façon avantageuse un effet de synergie.

L'association d'un extrait de sureau et d'une souche de *lactobacillus rhamnosus* selon l'invention a montré de bonnes capacités à induire et/ou stimuler une réponse immunitaire non spécifique démontrant ainsi son intérêt dans les domaines alimentaire et/ou pharmaceutique.

La composition peut avantageusement se présenter sous toutes les formes galéniques normalement utilisées dans les domaines alimentaire et/ou pharmaceutique pour une administration par voie orale ou sublinguale.

Dans le cas d'un aliment, il peut s'agir notamment de produits laitiers frais, de produits laitiers fermentés, de yaourts, de lait fermentés, de laits infantiles, de poudres, de pastilles, des jus végétaux, des boissons et de leurs mélanges, avantageusement dans le groupe constitué des produits laitiers frais, des jus de fruits et/ou de légumes ou des compotes de fruits.

De façon avantageuse, le fruit est choisi dans le groupe constitué par pomme, orange, fruits rouges, fraise, pêche, abricot, prune, framboise, mure, groseille, citron, pamplemousse, banane, ananas, kiwi, poire, cerise, noix de coco, fruits de la passion, mangue, figue, rhubarbe, melon, fruits exotiques, litchi, raisins, myrtille ou leurs mélanges.

Par « complément alimentaire », on entend au sens de la présente invention une denrée alimentaire dont le but est de compléter le régime alimentaire normal et qui constitue une source concentrée de nutriments ou d'autres substances ayant un effet nutritionnel ou physiologique seuls ou combinés.

Avantageusement, le complément alimentaire peut comprendre des vitamines et/ou des minéraux et/ou des oligoéléments.

Parmi ces vitamines, on peut en particulier citer, les vitamines A, C, D et E et les vitamines du groupe B, notamment, B1, B2, B3, B5, B6, B8, B9 et B12.

La vitamine A (rétinol ou caroténoïdes), la vitamine E et la vitamine C sont les 3 vitamines majeures antioxydantes. La vitamine A est présente uniquement dans les aliments d'origine animale. Néanmoins, certains végétaux contiennent des provitamines A, c'est à dire des substances que l'organisme est capable de transformer en vitamine A. Elle est indispensable à la vision et à la croissance des bronches, des intestins ou encore de la peau. La vitamine A intervient également dans les mécanismes immunitaires. Les substances antioxydantes s'opposent aux effets des radicaux libres en modérant leurs dommages, restaurant les lésions subies par les cellules, éliminant les déchets, entravant le phénomène d'oxydation. Lorsque les radicaux libres sont en excès dans l'organisme, ils peuvent favoriser le vieillissement cellulaire. Ces vitamines antioxydantes ont la capacité de contrôler la production des radicaux libres issus du métabolisme de l'oxygène, et dont la surproduction est responsable de très nombreux troubles, notamment au niveau cardio-vasculaire, immunité, cancer, cutané, vieillissement accéléré, maladies neuro-dégénératives, etc. En particulier, la vitamine c est un antioxydant bien connu, soluble dans l'eau. Les humains dépendent de sources externes de vitamine C pour satisfaire leurs besoins en vitamine C. Ainsi, l'acide ascorbique, l'ascorbate de sodium, ou leur mélange sont une source de vitamine C. La vitamine C est nécessaire à la synthèse des vaisseaux sanguins et des muscles. Elle favorise l'absorption du fer présent dans les aliments et intervient dans plusieurs mécanismes hormonaux. Elle joue également un rôle dans l'élimination des substances toxiques. Une déficience en vitamine C peut diminuer la résistance aux infections. La vitamine E est également un antioxydant bien connu. La vitamine E peut opérer en synergie avec la vitamine C afin de protéger la fonction cellulaire vitale contre les oxydants normaux. L'acétate d'alpha-tocophéryle, l'acétate de triméthyl-tocophéryle et/ou le succinate de vitamine E sont des sources de vitamine E. La vitamine E a un effet protecteur particulièrement important vis-vis des cellules de l'organisme. Elle joue un rôle important dans les mécanismes de la procréation et intervient dans la synthèse des globules rouges.

La vitamine D est désignée comme une vitamine liposoluble, bien que ce soit avant tout une hormone synthétisée dans l'organisme humain à partir d'un dérivé du cholestérol sous l'action des rayonnements UVB de la lumière. Elle existe sous 2 formes, D2 (ergocalciférol) ou D3 (cholécalciférol). Ces 2 molécules sont des 9,10-sécostéroïdes. La vitamine D intervient dans l'absorption du calcium et du phosphore par les intestins, ainsi que dans leur réabsorption par les reins, c'est une véritable hormone. D'autre part, elle influence plus de 200 gènes, ce qui explique son importance non soupçonnée jusque récemment dans de nombreuses maladies dont l'arthrite, les troubles de la peau apparentés au psoriasis, le diabète, certains cancers et même la démence.

Les vitamines B forment un groupe de vitamines hydrosolubles qui jouent un rôle important dans le métabolisme des cellules, parmi lesquelles on peut trouver :
- la vitamine B1 ou thiamine, qui est le cofacteur du complexe pyruvate décarboxylase ;
- La vitamine B2 ou riboflavine qui est le précurseur de la flavine adénine dinucléotide ;
- La vitamine B3 (PP) ou nicotinamide qui est le précurseur du nicotinamide adénine dinucléotide ;
- La vitamine B5 ou acide panthothénique qui est le précurseur et constituant du coenzyme A ;
- La vitamine B6 ou pyridoxine qui est le coenzyme des réactions liées au métabolisme des acides aminés et des protéines ;
- La vitamine B8 (H) ou biotine qui est le coenzyme des réactions de métabolisme des acides gras, des glucides et des acides aminés ;
- La vitamine B9 ou acide folique et la vitamine B12 ou cyanocobalamine qui sont des cofacteurs avec la S-adénosylméthionine.

Les vitamines B fonctionnent souvent ensemble au bénéfice de la santé du corps. Elles augmentent le métabolisme, aident à maintenir une peau saine et des muscles en bonne santé, améliorent le système immunitaire, améliorent le système nerveux et retardent les troubles cognitifs de la maladie d'Alzheimer, promeuvent la croissance des cellules ainsi que leur divisions, elles combattent les symptômes causés par un excès de stress.

Avantageusement, les minéraux sont choisis parmi le fer, le zinc, le sélénium, le chrome, le manganèse, le molybdène et leurs mélanges. Les sels minéraux sont des substances provenant de roches qui entrent dans la composition des organismes et qui sont présents dans l'alimentation animale et végétale. Ils se présentent sous forme ionique, on distingue les éléments minéraux majeurs, comme le calcium, le fer, le magnésium, le phosphore, le potassium, le sodium, le soufre et les oligoéléments comme l'aluminium, l'arsenic, le bore, le chlore, le chrome, le cobalt, le cuivre, le fluor, l'iode, le manganèse, le molybdène, le nickel, le plomb, le silicium, le sélénium, le vanadium, le zinc.

Le fer est l'un des sels minéraux essentiels au bon fonctionnement de l'organisme. Il a un rôle fondamental dans la constitution de l'hémoglobine contenue dans les globules rouges, dans la constitution de la myoglobine contenue dans les muscles et dans celle de nombreuses enzymes indispensables au fonctionnement de l'organisme. Sa carence est fréquente dans le monde entier, elle se traduit par une anémie, accompagnée d'une réduction de la capacité physique et intellectuelle, d'une diminution de la résistance aux infections.

Le zinc joue un rôle antioxydant en tant que cofacteur des enzymes qui participent directement à la défense contre les oxydants. L'oxyde de zinc, le gluconate de zinc, le citrate de zinc, l'acétate de zinc, le chlorure de zinc, le lactate de zinc ou le sulfate de zinc peuvent être utilisés seuls ou en mélange dans les compositions selon la présente invention. Avantageusement il s'agit du gluconate de zinc.

Le sélénium joue aussi un rôle antioxydant en tant que cofacteur des enzymes, notamment la glutathione peroxydase, qui participe directement à la défense contre les oxydants.

Le chrome est un oligo-élément qui intervient dans le métabolisme des glucides, il favorise l'action de l'insuline. Il intervient aussi dans le métabolisme des lipides et dans celui du cholestérol, il contribue à diminuer sa concentration. Le chrome joue aussi son rôle dans le métabolisme des acides nucléiques.

Le manganèse est un oligo-élément indispensable à l'efficacité de la vitamine B1. Il intervient aussi dans certaines métalloprotéines telles que la superoxyde dismutase. Il agit comme cofacteur de nombreuses enzymes. C'est aussi un métal essentiel pour la synthèse d'enzymes participant à la lutte contre le stress oxydant et qui préviennent des dommages causés par les radicaux libres.

Le molybdène intervient à de nombreux niveaux en tant que constituant de certaines enzymes, notamment la xanthine oxydase. Il joue un rôle dans la croissance osseuse et la structure de dents. Il a une action favorable sur le métabolisme du fer. Le molybdène participe aussi à la détoxication de l'organisme et il intervient dans la synthèse de l'acide urique.

Des principes actifs optionnels additionnels peuvent être ajoutés selon l'invention. Par exemple, le coenzyme Q10 et/ou un extrait de Ginseng. Le coenzyme Q10 ou ubiquinone est une substance similaire à une vitamine qui est vitale pour le fonctionnement du corps humain. Le coenzyme Q10 est naturellement présent dans toutes les cellules humaines et assure la production de l'énergie corporelle dans les mitochondries. Environ 95% des besoins corporels en énergie sont transformés à l'aide du coenzyme Q10. Cette substance ne peut être remplacée par aucune autre substance. Si on augmente par supplément alimentaire l'apport de coenzyme Q10, la capacité à l'exercice est renforcée. Le métabolisme lipidique est plus efficace, tout comme la consommation maximale en oxygène et le temps à l'effort. La réduction du taux de coenzyme Q10 est liée au vieillissement, mais également au stress.

Le ginseng est la plante médicinale qui jouit de la plus grande renommée en Asie. Le ginseng est avant tout un stimulant du système nerveux, physique et intellectuel et accroît la résistance physique. C'est un stimulant, vasomoteur qui a une action de fond sur l'organisme, il permet une meilleure résistance aux divers stress.

La composition peut être administrée par voie orale ou toute autre voie d'administration pharmaceutique.

Les compositions peuvent être formulées pour l'administration aux mammifères, y compris l'homme. Ces compositions sont réalisées de façon à pouvoir être administrées par voie orale, sublinguale, sous-cutanée, intramusculaire, intraveineuse, transdermique, locale ou rectale. Dans ce cas, les ingrédients actifs peuvent être administrés sous formes unitaires d'administration, en mélange avec des supports pharmaceutiques classiques, aux animaux ou aux être humains.

Dans le cas d'un complément alimentaire ou d'un médicament, les formes galéniques suivantes peuvent être envisagées : gélules, comprimés à avaler, comprimés à croquer, comprimés effervescents, pastilles, pilules, poudres, granules, les solutions ou suspensions orales et les formes d'administration sublinguale et buccale, les formes d'administration sous-cutanée, topique, intramusculaire, intraveineuse, intra nasale ou intraoculaire et les formes d'administration rectale. La forme galénique préférentielle est la gélule.

Lorsque l'on prépare une composition solide sous forme de comprimé, on mélange les ingrédients actifs avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant les ingrédients actifs avec un diluant (étape facultative) et en versant le mélange obtenu dans des gélules molles ou dures.

Une préparation sous forme de sirop ou d'élixir peut contenir les ingrédients actifs conjointement avec un édulcorant, un agent donnant du goût et un colorant approprié.

Les poudres ou les granules dispersibles dans l'eau peuvent contenir les ingrédients actifs en mélange avec des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire etc.), intra nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Les principes actifs peuvent être formulés également sous forme de microcapsules, éventuellement avec un ou plusieurs supports additifs.

Avantageusement, la composition n est destinée à une administration par voie orale.

La composition peut être utilsiée pour stimuler l'immunité et/ou renforcer les défenses immunitaires et/ou favoriser une réponse anti-infectieuse et/ou anti-inflammatoire et/ou au maintien de la vitalité.

Il est entendu que l'utilisation relative au maintien de la vitalité est fondée sur des allégations portées par les vitamines B2, B5, B6, B12, C et le fer. Ce sont des allégations santé autorisées par l'European Food Safety Authority (EFSA) sur ces nutriments. Elles s'appuient sur des données scientifiques qui mesurent les valeurs métaboliques de production d'énergie via l'utilisation des glucides, lipides ou protides. Cela impacte également la synthèse d'hormones qui agissent sur le métabolisme, comme les hormones stéroïdiennes.

Cette utilisation est donc soutenue par les références suivantes :
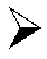 EFSA Journal 2009 ; 7(9) : 1215 Scientific opinion on the substantiation of health claims related to iron and formation of red blood cells and haemoglobin (ID 249, ID 1589), function of the immune system (ID 252, ID 259), cognitive function (ID 253) and cell division (ID 368) pursuant to Article 13(1) of regulation (EC) n° 1924/2006.
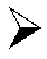 Huskisson E., Maggini S., Ruf M., Journal of International Medical Research 2007, 25 : 277-289.
   The role of vitamins and minerals in energy metabolism and well-being.
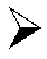 Institute of Medicine (1998), National Academy press, Washington, DC (592 pages). Dietary reference intakes for Thiamin, Riboflavin, Niacin, Bitamin B6, Folate, Vitamin B12, Pantothenic acid, Biotin, and Choline. A report of the stanfing committee on the scientific evaluation of dietary reference intakes and its panel of folate, other B vitamins, and choline and subcommittee on upper reference levels of nutrients food and nutrition board institute of medicine.

L'utilisation de la composition dans le traitement et/ou la prévention des infections respiratoires et/ou intestinales est également décrite.

L' utilisation dans le traitement et/ou la prévention des symptômes provoqués notamment par une infection par le virus de type 1 de l'herpès simplex, le virus respiratoire syncytial et les virus Influenza (A et/ou B et/ou C) et parainfluenza, avantageusement les symptômes des états grippaux, notamment provoqués par les virus Influenza (A et/ou B et/ou C) est également décrite.

La composition est particulièrement appropriée au traitement et/ou à la prévention des symptômes des états grippaux provoqués par une infection par des virus Influenza (*Orthomyxovirus, Influenzae* A, B ou C). Ces symptômes comprennent les rhumes, rhinites, toux, les inflammations de la muqueuse nasale (rhinorée, obstruction nasale, crise éternuements), maux de gorge (différentes manifestations : picotements, difficulté à déglutir), fièvre, maux de tête, courbatures, fatigues.

Ces « maux de l'hiver » sont associés à l'affaiblissement de nos défenses immunitaires.

L'utilisation de la composition pour la préparation d'un aliment, d'un complément alimentaire ou d'un médicament est également envisagée.

L'utilisation de la composition pour la préparation d'un médicament destiné au traitement et/ou à la prévention des infections respiratoires et/ou intestinales est également envisagée.

L'utilisation de la composition pour la préparation d'un médicament destiné au traitement et/ou à la prévention des symptômes une infection par le virus de type 1 de l'herpès simplex, le virus respiratoire syncytial et les virus Influenza (A et/ou B et/ou C) et parainfluenza, avantageusement les symptômes des états grippaux, notamment provoqués par les virus Influenza (A et/ou B et/ou C) est également décrite.

Un autre objet est l'utilisation de la composition pour la préparation d'un médicament pour son utilisation pour stimuler l'immunité et/ou favoriser une réponse anti-infectieuse et/ou anti-inflammatoire et/ou participer au maintien de la vitalité.

C'est enfin un autre objet que de fournir un procédé de renforcement des défenses immunitaires qui consiste en une administration par voie orale d'une composition à base d'une association d'un extrait de sureau et d'au moins une souche de *Lactobacillus rhamnosus.*

La composition est appropriée pour tous les âges de la vie. Elle est particulièrement adaptée chez le très jeune enfant. On entend par « très jeune enfant » au sens de la présente invention un enfant à partir de 6 mois et jusqu'à 3 ans. La composition convient tout particulièrement au « junior », on entend par « junior » au sens de la présente invention un enfant à partir de 3 ans et jusqu'à 17 ans. La composition est également particulièrement adaptée chez l'adulte, on entend par « adulte » au sens de la présente invention, une personne âgée de 18 à 60 ans. Mais la composition est également tout particulièrement appropriée chez le « sénior », on entend par « sénior » au sens de la présente invention, une personne âgée d'au moins 60 ans.

L'invention pourra être mieux comprise à l'aide des exemples non limitatifs qui suivent et qui constituent des modes de réalisation particuliers des compositions selon l'invention.

### Exemple 1 : préparation d'un extrait de sureau utilisé dans le cadre de la présente invention

L'extrait de sureau est obtenu à partir de jus de baies de sureau enrichi en anthocyanes et séché sur maltodextrine. L'enrichissement a été effectué par un procédé de filtration membranaire selon la méthode suivante : 26 litres de jus de sureau clarifié sont dilués avec 284 litres d'eau (11 volumes d'eau pour 1 volume de jus). Les 310 litres ainsi obtenus sont agités et chauffés à 45°C puis diafiltrés sur une première membrane de 5 kDa en rajoutant 7 volumes d'eau par volume de jus (soit 181 L). Le perméat de dialyse est ensuite concentré par ultrafiltration sur membrane de 5 kDa (membrane pilote de 5/10 kDa de surface unitaire 5.8 m²). Le retentât de l'ultrafiltration est concentré thermiquement sous vide à une température n'excédant pas 50°C, puis atomisé sur un support maltodextrine tel que la quantité de maltodextrine soit inférieure ou égale à 30% (p/p).

Les caractéristiques de l'extrait ainsi obtenu sont :
- Teneur en anthocyanes : 10-14% en poids par rapport à la matière sèche de l'extrait (exprimé en cyanidine-3-glucoside selon la méthode HPLC décrite par Wu et al., J. Agric. Food Chem. 52(26), 7846-7856, 2004).
- Teneur en protéines : 5-7% en poids par rapport à la matière sèche de l'extrait (N x 6,25 selon la méthode de Kjeldahl).

### Exemple 2 : complément alimentaire sous forme de gélule, cette composition est particulièrement appropriée pour l'adulte.

- Souche probiotique lyophilisée, *Lactobacillus rhamnosus* : 35 mg, soit 5.10⁸ UFC ;
- Extrait de sureau (*Sambucus nigra*) selon l'exemple 1: 50 mg ;
- Vitamine A : 400 µg ;
- Vitamine B1 : 550 µg ;
- Vitamine B2 : 700 µg ;
- Vitamine B5 : 3 mg ;
- Vitamine B6 : 700 µg ;
- Vitamine B8 : 25 µg ;
- Vitamine B9 : 100 µg ;
- Vitamine B12 : 0,625 µg ;
- Vitamine C : 40 mg ;
- Vitamine D : 2,5 µg ;
- Vitamine E : 6 mg ;
- Fer : 2,5 mg ;
- Zinc : 2,5 mg ;
- Sélénium : 15 µg ;
- Chrome : 12,5 µg ;
- Manganèse : 500 µg ;
- Molybdène : 12,5 µg.

### Exemple 3 : complément alimentaire sous forme de sachet, cette composition est particulièrement appropriée pour les très jeunes enfants.

- Souche probiotique lyophilisée, *Lactobacillus rhamnosus* : 35 mg, soit 5.10⁸ UFC ;
- Extrait de sureau (*Sambucus nigra*) selon l'exemple 1: 25 mg ;
- Vitamine C : 18 mg ;
- Vitamine B6 : 600 µg ;
- Vitamine E : 2,4 mg ;
- Zinc : 2,5 mg ;
- Sélénium : 15 µg.

### Exemple 4 : Etude in vitro des effets immuno-modulateurs combinés de la souche Lactobacillus rhamnosus avec un extrait de sureau dans des cellules mononuclées du sang périphérique Matériel et méthode

Des cellules mononucléées du sang périphérique sont isolées de sujets sains, remises en suspension et réparties dans des plaques à 24 puits à raison de 5 x 10⁵ cellules/puits. Ces cellules sont incubées pendant 18 h à 37°C sous 5% ce CO₂. Ces cellules sont stimulées soit avec 10 µg/ml de lipopolysaccharide, soit en présence de 100 µg/ml d'extrait de sureau, soit avec 5.10⁵ *Lactobacillus rhamnosus,* soit en présence de 100 µg/ml d'extrait de sureau et 5.10⁵ *Lactobacillus rhamnosus.* Les surnageants de culture sont récupérés pour doser les différentes cytokines par cytométrie en flux, ils ont été centrifugés à 1600 tr/min pendant 10 minutes à 4°C et conservés à -20°C jusqu'au dosage.

Le dosage des cytokines a été réalisé par la technique Luminex qui repose sur le principe de la cytométrie en flux. Cette technologie de Luminex est basée sur le principe du dosage ELISA en microplaque à 96 puits, avec comme support, des microbilles incorporant deux fluorochromes selon un ratio précis, ce qui leur confère un code couleur les identifiant (fluorescences différentes). Le système optique du cytomètre (Bio-Plex 200) est constitué de deux lasers : un laser rouge (λ = 635 nm) excite dans chaque microbille le mélange de colorants qui la définie, et identifie ainsi la cytokine à doser. Le second laser vert, (λ = 532 nm) excite le fluorochrome rapporteur attaché à l'anticorps spécifique de détection afin de quantifier la cytokine. Le système est piloté par un ordinateur muni d'un logiciel d'acquisition et d'analyse des données (Bio-Plex Manager version 4.1). Après décongélation, les surnageants ont été testés purs et dilués au 1/20^{ème} en milieu de culture, grâce à un kit Milliplex (Millipore, référence HCYTOMAG-60K-29). Celui-ci comprend les billes spécifiques, les anticorps de détection et les standards pour doser les cytokines suivantes : IL-1β, IL-1RA, IL-2, IL-4, IL-5, IL-6, IL-9, IL-10, IL-12p70, IL-13, IL-15, IL-17A, CCL2, CCL3, CCL4, CCL5, CCL22, CXCL8, CXCL10, TNFα, IFNγ, GM-CSF.

La concentration de chaque cytokine est exprimée en pg/ml, elle a été moyennée à partir des valeurs obtenues par 6 donneurs. Le seuil de détection de chaque cytokine est de 3,2 pg/ml. Pour les échantillons sous le seuil de détection, une valeur arbitraire correspondant au seuil de détection x ½, soit 1,6 pg/ml a été attribuée. L'analyse statistique a comparé le log₁₀ de la concentration de chaque cytokine par une ANOVA.

### Résultats

Les concentrations obtenues pour chaque condition de stimulation par cytokine, sont rassemblées dans les tableaux 1 et 2. Pour qu'il y ait synergie au niveau d'une cytokine, il faut que :
- la concentration de cytokine obtenue expérimentalement en stimulant les cellules mononucléées du sang périphérique simultanément avec le probiotique et l'extrait de sureau, soit supérieure à la somme des concentrations de cytokine observée individuellement avec le sureau et avec le probiotique ;
- la concentration médiane de cytokine obtenue avec l'association pour les 6 donneurs soit significativement supérieure à la fois à celle obtenue avec le sureau et celle obtenue avec le probiotique.

Dans le tableau 1 sont résumés les résultats de l'activité de l'extrait de sureau associé au probiotique *Lactobacillus rhamnosus* et la contribution de chacun des actifs sur la production de cytokines par des cellules mononucléées du sang périphérique humaines.

**TABLEAU 1**

| Cytokines | Effet sureau | Effet L. rhamnosus | Effet L. rhamnosus + sureau | Synergie |
|---|---|---|---|---|
| IL-1β | +/- | + | ++ | Non |
| IL-1RA | + | + | ++++ | Oui |
| IL-2 | - | - | - | Non |
| IL-4 | + | - | + | Non |
| IL-5 | - | - | - | Non |
| IL-6 | + | ++ | ++++ | Oui |
| IL-9 | - | - | - | Non |
| IL-10 | +/- | + | ++++ | Oui |
| IL-12p70 | - | + | + | Non |
| IL-13 | - | - | - | Non |
| IL-15 | - | - | - | Non |
| IL-17A | - | - | - | Non |
| CCL2 | ++ | +++ | +++ | Non |
| CCL3 | ++ | +++ | ++++ | Oui |
| CCL4 | ++ | +++ | ++++ | Oui |
| CCL5 | - | - | - | Non |
| CCL22 | +/- | + | ++ | Non |
| CXCL8 | ++ | +++ | ++++ | Oui |
| CXCL10 | ↓ | ++ | + | Non |
| TNFα | + | ++ | | Non |
| IFNγ | - | + | + | Non |
| GM-CSF | - | + | ++++ | Oui |

Une augmentation d'IL-1β en réponse au sureau n'est observée que pour 2 des 6 donneurs testés. Le probiotique déclenche une production d'IL-1β chez 5/6 des donneurs. L'association du sureau et probiotique augmente la production d'IL-1β, mais n'atteint pas la significativité statistique.

Tous les donneurs testés produisent de l'IL-IRA (antagoniste au récepteur de l'IL-1) en réponse au sureau, suggérant l'activité anti-inflammatoire du sureau. Tous les donneurs testés produisent de l'IL-IRA en réponse à *Lactobacillus rhamnosus*. La concentration de l'IL-IRA obtenue avec l'association sureau probiotique pour les 6 donneurs est significativement supérieure à la fois au sureau et au probiotique. De plus la concentration de l'IL-IRA obtenue avec l'association sureau probiotique est supérieure à la somme de celle obtenue par le probiotique et celle obtenue par le sureau, il ya donc synergie des 2 actifs pour la production d'IL-1RA.

Une augmentation d'IL-2 en réponse au sureau n'est observée que pour un seul des 6 donneurs testés. Trois des 6 donneurs répondent au probiotique par une production modérée d'IL-2. L'association des 2 actifs n'a pas d'impact sur la production d'IL-2.

Aucun des donneurs testés ne répond au sureau par une production d'IL-5. De la même façon, aucun des donneurs testés ne répond au probiotique par une production d'IL-5. L'association des 2 actifs n'a pas d'impact sur la production d'IL-5.

Tous les donneurs testés produisent de l'IL-6 en réponse au sureau. Cinq des 6 donneurs répondent au probiotique, il est à noter que le non-répondeur possède le plus fort taux basal d'IL-6. Pour les répondeurs, le probiotique est un plus puissant inducteur d'IL-6 que le sureau. L'association des 2 actifs provoque une production synergique d'IL-6.

Le tableau 2 montre un exemple de résultats de l'activité de l'extrait de sureau associé au probiotique *Lactobacillus rhamnosus* et la contribution de chacun des actifs sur la production d'IL-6 par des cellules mononucléées du sang périphérique humaine obtenus chez les 6 donneurs.

**TABLEAU 2**

| Groupes | Donneur 1 | Donneur 2 | Donneur 3 | Donneur 4 | Donneur 5 | Donneur 6 |
|---|---|---|---|---|---|---|
| témoin | 1,6 | 1,6 | 7 | 1,6 | 69 | 4 |
| Prob | 148 | 301 | 968 | 362 | 51 | 1148 |
| Sureau | 17 | 27 | 44 | 12 | 405 | 17 |
| Prob + sureau | 1258 | 1426 | 5398 | 1721 | 6081 | 5369 |

| | | | | | | |
|---|---|---|---|---|---|---|
| Prob : probiotique, *Lactobacillus rhamnosus* | | | | | | |

Aucun des donneurs testés ne répond au sureau par une production d'IL-9. De même, aucun des donneurs testés ne répond au probiotique par une production d'IL-9. L'association des 2 actifs n'a pas d'impact sur la production d'IL-9.

Quatre des 6 donneurs testés produisent de l'IL-10 en réponse au sureau. Tous les donneurs testés produisent de l'IL-10 en réponse au probiotique. La concentration d'IL-10 obtenue avec le sureau + le probiotique pour les 6 donneurs est significativement supérieure à la fois à celle obtenue avec le sureau et celle obtenue avec le probiotique. De plus, la concentration de l'IL-10 obtenue avec l'association sureau probiotique est supérieure à la somme de celle obtenue par le probiotique et celle obtenue par le sureau, il y a donc synergie des 2 actifs pour la production d'IL-10.

Aucun des donneurs testés ne répond au sureau par une production d'IL-12p70. En revanche, tous les donneurs produisent de l'IL-12p70 en réponse au probiotique. L'association du sureau et du probiotique ne potentialise pas la production d'IL-12p70.

Aucun des donneurs testés ne répond au sureau par une production d'IL-15. De même, aucun des donneurs testés ne répond au probiotique par une production d'IL-15. L'association des 2 actifs n'a pas d'impact sur la production d'IL-15.

Aucun des donneurs testés ne répond au sureau par une production d'IL-17A. Il en est de même pour la réponse au probiotique : il n'induit la production d'IL-17A chez aucun des donneurs. L'association des 2 actifs n'a pas d'impact sur la production d'IL-17A.

Tous les donneurs testés répondent au sureau par une production de CCL2. Cinq des 6 donneurs répondent au probiotique, il est à noter que le non-répondeur possède le plus fort taux basal de CCL2, le probiotique déclenche une réponse plus forte que le sureau. L'association des 2 actifs stimule une forte sécrétion de CCl2 significativement supérieure au sureau mais pas au probiotique.

Tous les donneurs testés répondent au sureau par une production de CCL3. Cinq des 6 donneurs répondent au probiotique, il est à noter que le non-répondeur possède le plus fort taux basal de CCL3, le probiotique déclenche une réponse plus forte que le sureau. La concentration de CCL3 obtenue avec le sureau + le probiotique pour les 6 donneurs est significativement supérieure à la fois à celle obtenue avec le sureau et celle obtenue avec le probiotique. De plus, la concentration de CCL3 obtenue avec l'association sureau probiotique est supérieure à la somme de celle obtenue par le probiotique et celle obtenue par le sureau, il y a donc synergie des 2 actifs pour la production de CCL3.

Tous les donneurs testés répondent au sureau par une production de CCL4. Tous les donneurs testés produisent CCL4 en réponse au probiotique, excepté pour un donneur qui présente un fort taux basal de CCL4, le probiotique déclenche une réponse plus forte que le sureau. La concentration de CCL4 obtenue avec le sureau + le probiotique pour les 6 donneurs est significativement supérieure à la fois à celle obtenue avec le sureau et celle obtenue avec le probiotique. De plus, la concentration de CCL4 obtenue avec l'association sureau probiotique est supérieure à la somme de celle obtenue par le probiotique et celle obtenue par le sureau, il y a donc synergie des 2 actifs pour la production de CCL4.

Aucun des donneurs testés ne répond au sureau par une production de CCL5. Un sel des 6 donneurs produit CCL5 en réponse au probiotique. L'association du sureau et du probiotique n'a pas d'action sur CCL5.

Seuls 2 des 6 donneurs testés produisent CCL22 en réponse au sureau. Tous les donneurs testés produisent CCL22 en réponse au probiotique. L'association des 2 actifs n'a pas d'action synergique sur CCL22.

Tous les donneurs testés produisent CXCL8 en réponse au sureau. Cinq des 6 donneurs répondent au probiotique, il est à noter que le non-répondeur possède le plus fort taux basal de CXCL8, le probiotique déclenche une réponse plus forte que le sureau. L'association des 2 actifs provoque une production synergique de CXCL8.

Aucun des donneurs testés ne produit de CXCL10 en réponse au sureau, à l'inverse le sureau inhibe significativement la production constitutive de CXCL10. Cinq des 6 donneurs répondent au probiotique. L'association des 2 actifs montre que le sureau inhibe significativement la production induite par le probiotique.

Tous les donneurs testés produisent du TNFα en réponse au sureau. Tous les donneurs testés produisent du TNFα en réponse au probiotique. Cette production est plus intense que celle déclenchée par le sureau. L'association du sureau et du probiotique n'a pas d'impact sur la production de TNFα.

Aucun des donneurs testés ne produit d'IFNy en réponse au sureau, en revanche, tous les donneurs testés produisent de l'IFNy en réponse au probiotique. L'association du sureau et du probiotique n'a pas d'impact sur la production d'IFNy.

Aucun des donneurs testés ne produit de GM-CSF en réponse au sureau. N revanche 5 des 6 donneurs produisent du GM-CSF en réponse au probiotique. La concentration de GM-CSF obtenue avec le sureau + le probiotique pour les 6 donneurs est significativement supérieure à la fois à celle obtenue avec le sureau et celle obtenue avec le probiotique. De plus, la concentration de GM-CSF obtenue avec l'association sureau probiotique est supérieure à la somme de celle obtenue par le probiotique et celle obtenue par le sureau, il y a donc synergie des 2 actifs pour la production de GM-CSF.

Le défaut de production d'IL-2, d'IL-5 et d'IL-9, des cytokines qui stimulent la prolifération des cellules T, suggère que l'association ne devrait pas activer l'expansion clonale des lymphocytes T.

La production d'IFNy et d'IL-12p70, typiques d'une réponse de type 1, sont dues à *Lactobacillus rhamnosus,* tandis que la modeste production d'IL-4, caractéristique d'une réponse de type 2, est liée à l'extrait de sureau. Le type de réponse est estimé par le ratio IFNy/IL-4 : la réponse est de type 1 pour un ratio supérieur à 100, de type 0 lorsque ce ratio est compris entre 0,01 et 100 et de type 2 s'il est inférieur à 0,01. Ce ratio est de 600 pour *Lactobacillus rhamnosus,* 3 pour l'extrait de sureau et 119 pour l'association sureau + *Lactobacillus rhamnosus,* qui est donc de type 1. La forte réponse de type 1 induite par le probiotique est donc bien contrebalancée par la production d'IL-4 déclenchée par le sureau.

Les productions d'IL-1β, IL-6, CCL2, CCL3, CCL4, CCL5, CCL22, CXCL8, TNFα et GM-CSF par l'association sureau et *Lactobacillus rhamnosus* suggèrent une activité inflammatoire avec recrutement et activation de cellules sur le site de l'inflammation. Etant donné les chemokines impliquées (CCL2, CCL3, CCL4, CCL5, CCL22, CXCL8), il y aura attraction préférentiellement de cellules de l'immunité innée, type polynucléaires et monocytes/macrophages, mais également de lymphocytes. Cette activité pro-inflammatoire sera modulée par la production d'IL-IRA, cytokine anti-inflammatoire qui se lie au même récepteur membranaire qu'IL-1, empêchant celle-ci d'envoyer son signal à la cellule. De plus, l'effet inhibiteur du sureau sur les productions constitutives et induites de CXCL10, chemokine attirant les lymphocytes activés, les cellules NK mais aussi les éosinophiles, va dans le même sens. L'activité pro-inflammatoire du probiotique semble donc être contrebalancée par l'effet anti-inflammatoire du sureau.

### Conclusions

Dans les conditions testées, les inventeurs ont montré que l'association d'un extrait de sureau et de *Lactobacillus rhamnosus* synergisent quant à l'induction d'IL-IRA, d'IL-6, d'IL-10, CCL3, CCL4, CXCL8, GM-CSF. Cette association induit la production d'IL-1b, IL-1RA, IL-4, IL-6, IL-10, IL-12p70, CCL2, CCL3, CCL4, CCL5, CCL22, CXCL8, IFNy, TNFα et GM-CSF. Ce profil suggère que l'association sureau + *Lactobacillus rhamnosus* stimule les défenses immunitaires en recrutant monocytes, polynucléaires et lymphocytes sur le site inflammatoire. Le profil de type 1 favorisera alors le développement de réponses de type cellulaire, notamment avec activation des monocytes/macrophages et neutrophiles, mais sans effet lymphoprolifératif. La réaction inflammatoire sera contenue par la production d'IL-IRA et d'IL-10, et par l'inhibition de production de CXCL10, liée spécifiquement au sureau.

## Revendications

1. Composition comprenant une association synergique d'un extrait de sureau et d'au moins une souche de *Lactobacillus rhamnosus* ledit extrait de sureau comprenant une teneur en anthocyanes comprise entre 0,5 et 25% en poids par rapport à la matière sèche de l'extrait et une teneur en protéines comprise entre 2 et 10% en poids par rapport à la matière sèche de l'extrait, pour son utilisation pour stimuler l'immunité et/ou renforcer les défenses immunitaires.

2. Composition comprenant une association synergique d'un extrait de sureau et d'au moins une souche de *Lactobacillus rhamnosus* ledit extrait de sureau comprenant une teneur en anthocyanes comprise entre 0,5 et 25% en poids par rapport à la matière sèche de l'extrait et une teneur en protéines comprise entre 2 et 10% en poids par rapport à la matière sèche de l'extrait, pour son utilisation dans le traitement et/ou la prévention des symptômes des états grippaux.

3. Composition pour son utilisation selon l'une des revendications 1 ou 2, **caractérisée en ce qu'**elle comprend une quantité de *Lactobacillus rhamnosus* comprise entre 1.10⁷ à 1.10¹¹ UFC/dose unitaire.

4. Composition pour son utilisation selon l'une quelconque des revendications 1 à 3, **caractérisée en ce qu'**il s'agit d'un complément alimentaire ou d'un médicament.

5. Composition pour son utilisation selon l'une quelconque des revendications 1 à 4, **caractérisée en ce qu'**il s'agit d'un complément alimentaire sous une forme galénique choisie dans le groupe constitué par les gélules, les comprimés à avaler, les comprimés à croquer, les comprimés effervescents, les pastilles, les poudres, les granules, les solutions ou suspensions orales et les formes d'administration sublinguale et buccale.

6. Composition pour son utilisation selon l'une quelconque des revendications 1 à 5, **caractérisée en ce qu'**elle comprend entre 20 mg et 200 mg d'extrait sec de sureau par dose unitaire, préférentiellement entre 20 mg et 100 mg d'extrait sec de sureau par dose unitaire, et entre 1.10⁷ et 1.10¹¹ UFC de *Lactobacillus rhamnosus* par dose unitaire, préférentiellement entre 1.10⁸ et 1.10⁹ UFC de *Lactobacillus rhamnosus* par dose unitaire.

7. Composition pour son utilisation selon l'une quelconque des revendications 1 à 6, **caractérisée en ce qu'**elle comprend en outre des vitamines et/ou des sels minéraux.

## Patentansprüche

1. Zusammensetzung, umfassend eine synergetische Verbindung aus einem Holunderextrakt und wenigstens einem Stamm *Lactobacillus Rhamnosus*, wobei der genannte Holunderextrakt einen zwischen 0,5 und 25 Gew.-% inbegriffenen Gehalt an Anthocyanen im Verhältnis zur Trockenmasse des Extrakts und einen zwischen 2 und 10 Gew.-% inbegriffenen Proteingehalt im Verhältnis zur Trockenmasse des Extrakts für seine Verwendung zur Stimulation der Immunität und / oder zur Verstärkung der Immunabwehr umfasst.

2. Zusammensetzung, umfassend eine synergetische Verbindung aus einem Holunderextrakt und wenigstens einem Stamm *Lactobacillus Rhamnosus*, wobei der genannte Holunderextrakt einen zwischen 0,5 und 25 Gew.-% inbegriffenen Inhalt an Anthocyanen im Verhältnis zur Trockenmasse des Extrakts und einen zwischen 2 und 10 Gew.-% an Proteinen im Verhältnis zur Trockenmasse des Extrakts für seine Verwendung bei der Behandlung und / oder der Vorbeugung von Symptomen von Grippeerkrankungen umfasst.

3. Zusammensetzung für ihre Verwendung gemäß einem der Ansprüche 1 oder 2, **dadurch gekennzeichnet, dass** sie eine zwischen 1,10⁷ und 1,10¹¹ inbegriffene UFC / Einheitsdosis inbegriffene Menge an *Lactobacillus Rhamnosus* umfasst.

4. Zusammensetzung für ihre Verwendung gemäß irgendeinem der Ansprüche 1 bis 3, **dadurch gekennzeichnet, dass** es sich um einen Lebensmittelzusatz oder ein Medikament handelt.

5. Zusammensetzung für ihre Verwendung gemäß irgendeinem der Ansprüche 1 bis 4, **dadurch gekennzeichnet, dass** es sich um einen Lebensmittelzusatz in einer galenischen Form handelt, die aus der Gruppe ausgewählt ist, die durch Kapseln, zu verschluckende Tabletten, Kautabletten, Brausetabletten, Pastillen, Pulver, Körnchen, Lösungen oder orale Suspensionen und die Formen zur sublingualen und bukkalen Verabreichung ausgewählt sind.

6. Zusammensetzung für ihre Verwendung gemäß irgendeinem der Ansprüche 1 bis 5, **dadurch gekennzeichnet, dass** sie zwischen 20 mg und 200 mg Holunder-Trockenextrakt pro Einheitsdosis, bevorzugt zwischen 20 mg und 100 mg Holunder-Trockenextrakt pro Einheitsdosis und zwischen 1,10⁷ und 1,10¹¹ UFC *Lactobacillus Rhamnosus* pro Einheitsdosis, bevorzugt zwischen 1,10⁸ und 1,10⁹ UFC *Lactobacillus Rhamnosus* pro Einheitsdosis umfasst.

7. Zusammensetzung für ihre Verwendung gemäß irgendeinem der Ansprüche 1 bis 6, **dadurch gekennzeichnet, dass** sie darüber hinaus Vitamine und / oder Mineralsalze umfasst.

## Claims

1. Composition comprising a synergistic combination of an elder extract and at least one strain of *Lactobacillus rhamnosus* said elder extract comprising an anthocyanin content comprised between 0.5 and 25% by weight compared to the dry matter of the extract and a protein content comprised between 2 and 10% by weight compared to the dry matter of the extract, for use in stimulating immunity and/or strengthening immune defences.

2. Composition comprising a synergistic combination of an elder extract and at least one strain of *Lactobacillus rhamnosus* said elder extract comprising an anthocyanin content comprised between 0.5 and 25% by weight compared to the dry matter of the extract and a protein content comprised between 2 and 10% by weight compared to the dry matter of the extract, for use in the treatment and/or prevention of flu-like states.

3. Composition for use thereof according to one of claims 1 or 2, **characterised in that** it comprises a quantity of *Lactobacillus rhamnosus* comprised between 1.10⁷ to 1.10¹¹ CFU/unit dose.

4. Composition for use thereof according to any one of claims 1 to 3, **characterised in that** it is a food supplement or a medicine.

5. Composition for use thereof according to any of claims 1 to 4, **characterised in that** it is a food supplement in a dosage form chosen from the group constituted of capsules, tablets to swallow, tablets to chew, effervescent tablets, pastilles, powders, granules, oral solutions or suspensions and sublingual and buccal administration forms.

6. Composition for use thereof according to any one of claims 1 to 5, **characterised in that** it comprises between 20 mg and 200 mg of dry extract of elder per unit dose, preferentially between 20 mg and 100 mg of dry extract of elder per unit dose, and between 1.10⁷ and 1.10¹¹ CFU of *Lactobacillus rhamnosus* per unit dose, preferentially between 1.10⁸ and 1.10⁹ CFU of *Lactobacillus rhamnosus* per unit dose.

7. Composition for use thereof according to any one of claims 1 to 6, **characterised in that** it further comprises vitamins and/or mineral salts.
